# EUROPÄISCHE PATENTSCHRIFT

(11) **EP 3 099 277 B1**
(45) Veröffentlichungstag und Bekanntmachung des Hinweises auf die Patenterteilung: **15.01.2020**
(21) Anmeldenummer: 15710092.6
(22) Anmeldetag: 26.01.2015
(51) Int. Cl.: A61F 7/10, F25D 3/08

(54) **KÜHLMATTE MIT DEHNELEMENTEN ZUM KÜHLEN EINES KÖRPERS ODER EINES KÖRPERTEILS FÜR MEDIZINISCHE ODER LEISTUNGSSTEIGERNDE ZWECKE (COOLAPP)**
COOLING MAT COMPRISING EXPANDABLE ELEMENTS FOR COOLING A BODY OR BODY PART FOR MEDICAL OR PERFORMANCE-ENHANCING PURPOSES (COOL APP)
TAPIS DE REFROIDISSEMENT COMPRENANT DES ÉLÉMENTS EXTENSIBLES POUR LE REFROIDISSEMENT D'UN CORPS OU D'UNE PARTIE D'UN CORPS À DES FINS MÉDICALES OU D'AMÉLIORATION DES PERFORMANCES (COOLAPP)

(30) Priorität: 30.01.2014 AT 682014
(43) Veröffentlichungstag der Anmeldung: 07.12.2016
(73) Patentinhaber: Vogel, Friedrich, 2500 Baden (AT); Vogel, Jakob, 2500 Baden (AT); Faworka, Rudolf, 1110 Wien (AT)
(72) Erfinder: Vogel, Friedrich, 2500 Baden (AT); Vogel, Jakob, 2500 Baden (AT); Faworka, Rudolf, 1110 Wien (AT)
(74) Vertreter: Babeluk, Michael
(86) Internationale Anmeldenummer: PCT/AT2015/000007
(87) Internationale Veröffentlichungsnummer: WO 2015/113079

(56) Entgegenhaltungen:
- EP-A2- 0 672 400
- WO-A1-2013/093917
- US-A- 1 941 173
- US-A- 5 897 582
- US-A1- 2002 035 745

## Beschreibung

Die Erfindung betrifft eine Vorrichtung zum Kühlen eines Körpers oder eines Körperteils durch eine wärmeentziehende Auflage.
Derartige Vorrichtungen dienen vorzugsweise zum Temperaturmanagement von Menschen oder Tieren die während oder nach Herzstillstand, Herzinfarkt, Schlaganfall, septischem Schock, Apoplexie, Rückenmarksverletzungen oder Schädel/Hirn Trauma eine Abkühlung der Körperkerntemperatur auf unter 36°C benötigen (therapeutische Hypothermie).

Auch nach erfolgreicher Wiederbelebung nach Herzstillstand ist die langfristige Überlebenschance und Qualität des Überlebens häufig gering. Rund 55% der Patienten versterben auch nach erfolgreicher Reanimation an den Spätfolgen eines Herzstillstandes innerhalb von 12 Monaten. Ein großer Teil der Überlebenden erleidet schwere Schädigungen von Gehirn und Nervensystem wodurch die Lebensqualität massiv beeinträchtigt wird. Dabei entsteht die Schädigung nicht nur während des Herzstillstands sondern schreitet auch nach erfolgreicher Wiederbelebung fort. Diese Schädigung nennt man Post-Reanimations-Syndrom. Untersuchungen haben gezeigt, dass die Überlebenschance und Überlebensqualität von Patienten mit Herzstillstand nach erfolgreicher Wiederbelebung durch rasche Absenkung der Körpertemperatur auf rund 33°C für die Dauer von 24 bis 48 Stunden deutlich verbessert werden kann. Durch diese, in der Medizin als therapeutische Hypothermie bezeichnete, Abkühlung wird nicht nur der Sauerstoffverbrauch im Gehirn des Patienten reduziert, sondern es werden verschiedene zelluläre Zerfallsprozesse unterdrückt oder verlangsamt. Dadurch wird körpereigenen Reparaturprozessen Zeit gegeben. In der Folge steigt die Überlebenswahrscheinlichkeit und Überlebensqualität signifikant. Weitere Anwendungsgebiete einer solchen effizient kühlenden Auflage finden sich im Bereich der Orthopädie, der Zahnmedizin und immer dann, wenn eine Schwellung nach einer Verletzung oder im postoperativen Bereich vermieden werden soll.

Studien weisen darauf hin, dass ein Einsatz bei Patienten nach Schlaganfall ebenfalls einen besseren Krankheitsverlauf bewirken kann. Die Anwendung von Kälte erfolgt auch bei rheumatischen Erscheinungsbildern und sonstigen entzündlichen Prozessen.

Die Anwendung der Kühlung am Menschen vor oder während des Sports kann nach einer Studie der Universität Münster auch zur Leistungssteigerung angewendet werden.

Momentan erhältliche, nichtinvasive Kühlungsvorrichtungen benötigen zur Aufbereitung der Kälteenergie meist Kühlaggregate, die die Kälte während der Kühlung, meist aus elektrischer Energie erzeugen und das kalte Medium flüssig oder gasförmig über den gesamten Körper oder Teile des Körpers zur Ableitung der Energie über Matten oder Berieselungsanlagen verteilen. Invasive Methoden benötigen ebenfalls, während oder vor der Anwendung hohe Energiemengen, die durch Umwandlung meist elektrischer Energie in Kälteaggregaten bereitgestellt wird.

Die US Patentanmeldung 11/432,285, publiziert als US20060276552, stellt ein halbinvasives Kühlsystem dar. Dabei wird ein leicht flüchtiges Gas, insbesondere Perfluorkarbon, in die Nasenhöhle eingeblasen. Durch die bei der Expansion des Gases entstehende Verdunstungskälte wird der Nasenschleimhaut Energie entzogen und dadurch der Blutstrom gekühlt. Dabei besteht jedoch durch die direkte Einwirkung des Gases auf die Gefäße und Nasenschleimhaut ein großes Risiko von Erfrierungen des Gesichts.

Bekannte Kühlvorrichtungen ohne externe Vorrichtung zur Durchströmung mit Flüssigkeit oder Gas sind als Kühlmatten ausgeführt. Einzelne Teile einer Kühlmatte, die der Energiespeicherung dienen, werden im Folgenden als Kühlkörper bezeichnet. Die Kühlkörper sind dabei mit kalter Flüssigkeit, Gel, oder Eis gefüllt und mit Klebefolien, Riemen oder Klettbändern versehen um eine gute Kontaktierung und damit einen guten Wärmeübergang zwischen Kühlkörper und dem zu kühlenden Patienten zu gewährleisten. Bei Erwärmung der einzelnen Kühlkörper wird die jeweilige Grenzschicht des Kühlkörpers erwärmt. Dadurch verringert sich die Kühlleistung im Zeitverlauf durch die absinkende Wärmeübertragung deutlich, auch wenn noch ausreichende Kühlkapazität im Kühlkörper selbst verfügbar wäre. Da beispielsweise Wasser eine geringe Wärmeleitfähigkeit von 0,597 W/(m·K) (bei20 °C) aufweist, sinkt die Wärmeübertragung zwischen dem festen Anteil eines eisförmigen Kühlkörpers und der Patientenoberfläche durch die an der Grenzschicht entstehende Flüssigkeitsbarriere in sehr kurzer Zeit und wird damit ineffizient. US Patent US 2010211143 A1 beschreibt eine Vorrichtung zum Anpressen von Kühlkörpern mit Hilfe von überlappenden Druckkammern welche über eine Einheit zur Druckadjustierung verfügt. Dieser und allen bekannten Vorrichtungen gemeinsam ist der Nachteil, dass der Kühlkörper jeweils in seiner Gesamtheit an die zu kühlende Fläche angepresst wird, die Bildung einer isolierenden Schicht wie zuvor beschrieben damit jedoch nicht verhindert werden kann. US Patent US 2009036960 beschreibt eine Umhüllung von Eis deren flüssiger Anteil beim Schmelzen durch Polymerkristalle aufgenommen wird. Nachteil dieser Vorrichtung ist, auch wenn damit eine isolierende Flüssigkeitsschicht durch die Schwerkraft abgeführt wird, dass die Befüllung der Auflage bereits mit gefrorenem Material erfolgen muss, da andernfalls die eingeführte Flüssigkeit sofort von den Polymerkristallen aufgenommen wird. Eine derartige Vorrichtung ist daher für die medizinische Nutzung im klinischen Bereich ungeeignet, da die Befüllung einer Auflage mit Eis sehr arbeitsaufwändig ist und eine, auch nur temporäre, Erwärmung die Vorrichtung zerstört, Ein weiterer Nachteil dieser Vorrichtung ist, das Eis in zermahlener Form eingebracht werden muss um eine Flexibilität zur Anpassung an die Körperform zu ermöglichen. Dadurch enthält der Kühlkörper zwangsläufig einen großen Luftanteil der wiederum durch Isolation die Effizienz der Kühlung verringert. Ein weiterer Nachteil der in diesem Patent beschriebenen Vorrichtung ist, dass die Abfuhr der Flüssigkeitsschicht lageabhängig ist und nicht unabhängig von der Schwerkraftrichtung erfolgt. Damit ist diese Vorrichtung nicht für eine Ganzkörperkühlung geeignet, bei der die Anordnung der Kühlkörper in Relation zur Sammelvorrichtung für die Flüssigkeit beliebig sein sollte.

Das Patent AT 414 094 B beschreibt eine Auflage, welche vor Anwendung unter den Gefrierpunkt abgekühlt wird und bei der Kühlung die freiwerdende Schmelzenergie zur raschen Abkühlung des Patienten nutzt. Dabei wird zur Verhinderung der Reduktion der Wärmeleitung zwischen Patienten und dem noch festen Kühlkörper durch die geschmolzene Flüssigkeit eine Verbesserung der Leitfähigkeit der Flüssigkeit durch das Einbringen von wärmeleitfähigen Materialien erzielt. Die Einbringung dieser Materialien ist jedoch produktionstechnisch komplex bzw. sind Materialen wie Grafit bei einem Austritt der Flüssigkeit aus dem Kühlkörper in Folge einer Beschädigung der Hülle stark verschmutzend. Neben der Irritation des medizinischen Personals durch die starke Färbewirkung des dabei verwendeten Materials Grafit besteht bei Materialaustritt auch das Risiko einer Beeinflussung der elektrischen Leitfähigkeit und damit Beeinflussung von medizinischen Instrumenten wie beispielsweise dem EKG. Ebenfalls erhöhen diese Beimengungen oder Beilagen das spezifische Gewicht bezogen auf die Kühlleistung.

Eine weitere wichtige Eigenschaft von Kühlauflagen ist es, die jeweilige Temperatur der Kühlauflage anzuzeigen. Damit wird dem behandelnden medizinischen Personal oder dem sonstigen Anwender signalisiert, dass die Kühlauflage noch ausreichend kühlt oder ausgewechselt werden muss. Herkömmliche Kühlauflagen haben die Indikatoren meist in Form von Thermochromfarben an der Oberfläche positioniert. Speziell bei Kühlkörpern die die Schmelzenergie zur Abkühlung nutzen sind die Temperaturunterschiede innerhalb eines Kühlkörpers selbst sehr hoch. Randschichten können bereits Raum- oder Körpertemperatur erreichen obwohl noch ausreichend Schmelzenergie in den festen Bestandteilen vorliegt. Eine Positionierung des Temperaturindikators auf der Oberfläche eines Kühl- oder Wärmekörpers oder an undefinierter Stelle im Flüssigkeits-Eis Gemisch führt daher je nach Situation zu vorzeitiger oder zu später Entfernung und Austausch der Kühlauflagen. Da Kühlauflagen für medizinische Anwendungen hohe Kosten verursachen kann eine genauere Temperaturindikation wesentliche. Einsparungsmöglichkeiten und verbesserte Sicherheit für den Patienten bieten.

US5575812 Patent beschreibt eine Kühl- oder Wärmeauflage mit an der Außenhaut des Kühlkörpers positioniertem Temperaturindikator an der Oberfläche in Form einer Schrift aus Thermochromfarben. Diese Art der Temperaturindikation an der Oberfläche ist für gelförmige oder flüssige Kühlkörper kleiner Ausdehnung meist ausreichend genau. Für Kühlkörper mit höherer Speicherkapazität, die auch die Schmelzenergie nutzen ist sie jedoch sehr ungenau. Die vom Indikator angezeigte Temperatur kann dabei von der Temperatur des noch festen Bestandteils des Kühlkörpers um bis zu 10 Grad in jede Richtung abweichen. So kann ein dem Tiefkühlschrank entnommenes Kühlelement an der Oberfläche bereits einen für die Kühlung sicheren Temperaturbereich signalisieren obwohl die dem Patienten zugewandte Seite noch eine hautschädigende Temperatur aufweist. Durch diese Fehlanzeige kann es zu schweren Verletzungen des bei der Therapie meist bewusstlosen und daher schmerzunempfindlichen Patienten kommen. Umgekehrt kann die, von einem an der Oberfläche angebrachten Thermoindikator angezeigte Temperatur bereits Raumtemperatur und damit Wirkungslosigkeit für Zwecke der Kühlung signalisieren, während die dem Patienten zugewandten noch festen Bestandteile noch eine aktive Kühlung ermöglichen. Ein verfrühter Austausch der Kühlmatte stellt jedoch, sowohl in Hinblick auf Arbeitsbelastung des medizinischen Personals, als auch in Hinblick auf die Kosten einer zusätzlichen Kühlmatte einen wesentlichen Nachteil dar. In US1941173 wird eine Kühlvorrichtung nach dem Oberbegriff des Anspruchs 1 offenbart.

Ausgehend vom Stand der Technik liegt der vorliegenden Erfindung die Aufgabe zugrunde, eine Auflage zum Kühlen zumindest von Teilen des Körpers von Menschen und Tieren zu schaffen, mit der hohe Kühlraten einfach, umweltverträglich und effizient erzielt werden können. Das Kühlsystem soll möglichst klein und leicht sein und während der Anwendung ohne externe Energiequelle Einsatzfähig sein, so dass ein Einsatz auch mobil ermöglicht wird. Des Weiteren soll die Kühlauflage ohne Beimengung von Materialien zur Erhöhung der Wärmeleitfähigkeit arbeiten um deren oben beschriebenen Nachteile zu überwinden. Die Temperaturindikation soll zuverlässig und mit hoher Genauigkeit die Temperatur des verbleibenden festen Anteils eines Kühlkörpers an der Grenzfläche zwischen Patient und Kühlkörper wiedergeben. Die dargelegten Nachteile bekannter Systeme sollen vermieden bzw. reduziert werden.

Die erfindungsgemäßen Aufgaben werden durch die kennzeichnenden Merkmale der Ansprüche 1 bis 3 gelöst. Die erfindungsgemäße Auflage dient dabei vorzugsweise zur Temperaturregelung zumindest von Teilen des Körpers von Menschen oder Tieren, wann immer dies zu medizinischen, therapeutischen, leistungsverbessernden oder komfortverbessernden Zwecken erforderlich ist. Die Kühlung erfolgt dabei mit Kühlkörpern in Form eines schmelzfähigen Materials, wie vorzugsweise Wasser, welche in einer Kühlauflage bestehend aus vorzugsweise kleinen luftfreien Einzelzellen in noch flüssiger Form eingebracht wird und vor der Anwendung in einem Tiefkühlschrank vorzugsweise bei Temperaturen von rund - 6°C eingefroren wird. Zur Anwendung wird die Kühlauflage auf den Körper- oder den Körperteil mittels einer Klebeschicht oder einer mechanischen Vorrichtung aufgebracht. Dabei wird für den Kühlvorgang die Schmelzenergie wirksam. Wird nun diese Kühlauflage mit dem zu kühlenden Körper oder Körperteil in Verbindung gebracht, beginnt der Kühlkörper zu schmelzen. Ohne eine besondere Vorrichtung würde die Kühlleistung nach wenigen Minuten deutlich absinken, da an der Grenzschicht zwischen dem Körper und dem noch festen Anteil in der Kühlauflage eine Flüssigkeitsschicht entsteht, welche auf Grund der schlechten Wärmeleitfähigkeit der meisten Flüssigkeiten bzw. von Wasser einen Wärmeisolator bildet. In der Folge kann die Energie an der Kontaktfläche nur mehr sehr langsam übertragen werden und die gewünschte schnelle Abkühlung des Körpers und damit des Patienten erfolgt nicht. Die im Kühlkörper gespeicherte "negative" Energie als Energiesenke würde dann die für den Schmelzvorgang benötigte Energie aus der Umgebung entziehen.

Die erfindungsgemäße Vorrichtung zum Kühlen eines Körpers oder eines Körperteils für medizinische oder leistungssteigernde Zwecke durch eine mittels Schmelzwärme einer Kühlmasse wärmeentziehende Auflage ist dadurch gekennzeichnet, dass der bereits geschmolzene Anteil der Kühlmasse zwischen dem noch festen Anteil der Kühlmasse und der Kontaktfläche zum Körper bzw. Körperteil ständig aktiv und lageunabhängig abgeführt wird. Bei einer Schmelzwärme des Wassers von 0,334 MJ/kg und einer spezifischen Wärmekapazität des Menschen von 0,035 MJ/kg kann die Abkühlung eines Körpers mit 80kg um rund 3°C unter Annahme eines 70% Wirkungsgrades des Gesamtsystems zur Kühlung mit 3,6 kg Eis erfolgen. Die Temperaturabsenkung um 3°C wird in der therapeutischen Hypothermie empfohlen.

Um die gute Wärmeleitfähigkeit zwischen dem festen Kühlkörper und der Hautoberfläche während des gesamten Schmelzvorgangs aufrecht zu erhalten wird erfindungsgemäß eine Vorrichtung zum selektiven Anpressen des noch festen Anteils eines Kühlkörpers auf einen zu kühlenden Körperteil und zur Verdrängung der flüssigen Anteile an der Grenzschicht zum zu kühlenden Körperteil, dadurch gekennzeichnet, dass in einer vorzugsweise quaderförmigen Kühleinheit ein vorzugsweise zentral positioniertes Dehnelement einen gerichteten Druck auf den noch festen Anteile des Kühlkörpers ausübt angebracht. Um eine gute Anpassung der, zu einer Kühlmatte verbundenen, Kühlkörper an die Körperoberfläche ermöglichen und ein Zerbrechen der festen Anteile innerhalb eines einzeln Kühlkörpers zu vermeiden, werden die einzelnen Kühlkörper dabei vorzugsweise in einer Größe von rund 30 x 30 mm Grundfläche ausgeführt. Während des Einfrierens wird die Kühlauflage in einer formstabilen Überhülle aufbewahrt, welche Vorzugsweise auch als Verpackung dient. Dehnt sich nun das Wasser oder die sonstige Kühlflüssigkeit beim Einfrieren aus, so werden die erfindungsgemäßen Dehnelemente komprimiert. Wird nun die Kühlauflage am Körperteil durch Kleben oder sonstige mechanische Vorrichtungen appliziert so schmilzt der feste Kühlkörper. Die dabei entstehende Flüssigkeit wird nun durch den Druck, den die komprimierten Dehnelemente auf den noch festen Anteil des Kühlkörpers ausüben von der Kontaktfläche zwischen festem Anteil des Kühlkörpers und dem zu kühlenden Körperteil verdrängt. Damit kann die Kühlleistung während des gesamten Schmelzvorganges weitgehend konstant gehalten werden. Dabei kann der Dehnkörper sowohl gasförmig oder aber auch als Festkörper bspw. in Form von Paraffinen ausgebildet werden.

Eine weitere Ausprägung der Vorrichtung zur Entfernung der isolierenden Flüssigkeitsschicht besteht in der Nutzung eines Dehnkörpers in Form einer kleinen Federelements oder eines dauerelastischen Materials wie beispielsweise Schaumstoff.

Die Außenhülle des Kühlkörpers und des darüber liegenden Dehnkörpers ist vorzugsweise aus Thermoplastkunststoff ausgeführt, wodurch sowohl die Reißfestigkeit, Verarbeitbarkeit, als auch die Anforderungen an die Dichtheit und Biokompatibilität des Materials erfüllt werden können.

Der Vorrichtung zur Temperaturindikation auf Thermochromfarbbasis ist erfindungsgemäß im Unterschied zu herkömmlichen Temperaturindikatoren im Inneren des Kühlkörpers, an der Unterseite des erfindungsgemäßen Dehnkörpers positioniert. Der Dehnkörper wird, sofern ein Temperaturindikator eingesetzt wird aus blickdurchlässiger Folie ausgeführt wodurch die Durchsicht durch den Dehnkörper möglich ist. Die Anbringung in diesem Bereich in Zusammenhang mit der Nutzung eines Dehnkörpers ermöglicht im Gegensatz zu herkömmlichen Temperaturindikatoren eine genaue Bestimmung einer Grenzwerttemperatur des noch festen Kühlkörpers, welche wiederum mit der Temperatur der Grenzschicht zum Körper weitgehend übereinstimmt. Dadurch kann der Anwender auch während der Behandlung einfach erkennen ob die Kühlauflage noch ausreichend Kühlleistung liefert oder ausgetauscht werden sollte. Diese Vorrichtung zur Temperaturschwellwert-Anzeige ermöglicht eine bedeutende Kosteneinsparung und erhöhte Sicherheit für den Patienten.

Die vorliegende Erfindung wird an Hand von Ausführungsbeispielen gemäß den beigefügten Zeichnungen näher erläutert:

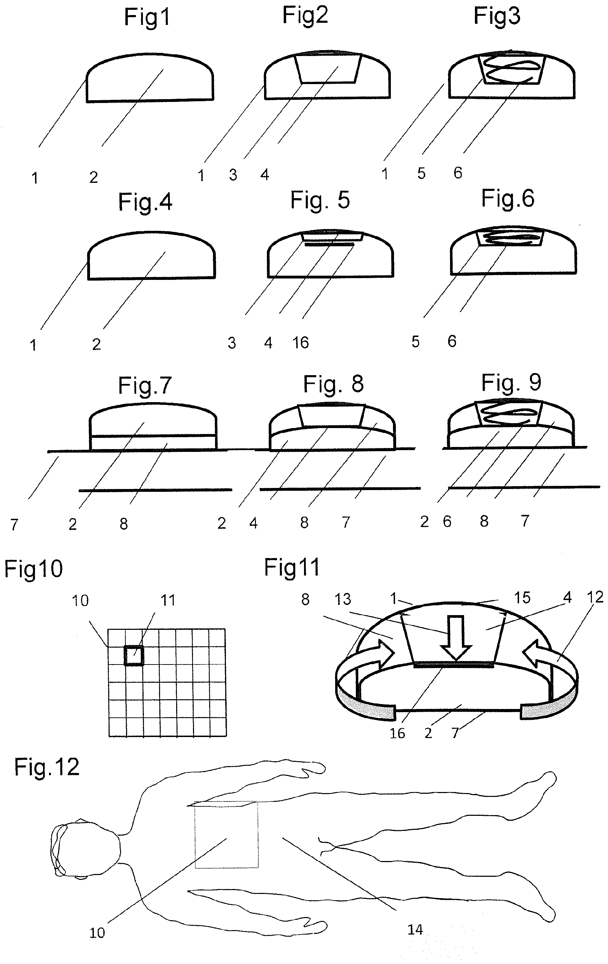

Fig1, 2 und 3 zeigen jeweils einen schematischen Schnitt durch ein Element einer Kühlauflage. Jedes dieser Elemente wird durch eine Hülle 1 und einen Kühlkörper 2, welcher aus einer Flüssigkeit - vorzugsweise Wasser - besteht, gebildet. Die Flüssigkeit soll dabei bei einer Temperatur von rund +10°C bis -10°C in den festen Aggregatszustand übergehen. Die Elemente werden in der Folge als Kühlelemente bezeichnet. Das Kühlelement der Fig1 ist dabei ohne erfindungsgemäße Vorrichtung abgebildet und enthält nur den Kühlkörper 2. Fig2 zeigt ein Kühlelement mit erfindungsgemäß im Kühlelement 1 integrierten, gasförmigen oder festen Dehnkörper 4, welcher mit einer elastischen Folie 3 umhüllt ist. Fig3 zeigt eine erfindungsgemäß mechanische Vorrichtung in Form einer Feder oder eines anderen elastischen Materials 6, welches ebenfalls durch eine elastische Hülle 5 vom Kühlkörper 1 getrennt ist.
Fig4, 5, und 6 zeigen die Kühlelemente aus Fig1, 2 und 3 im gefrorenen Zustand. Die Hülle 1 ist dabei in allen Fällen so elastisch, dass Sie die Ausdehnung des Kühlkörpers 2 beim Einfrieren absorbieren kann ohne zu reißen, dabei jedoch eine Spannung entsteht, welche die Dehnkörper 4 in Fig5 oder das elastische Material wie beispielsweise eine Feder 6 in Fig6 zusammenpresst. Die Hüllen der Dehnkörper in Fig5 und 6 sind dabei elastischer als Hülle 1.
Fig7, 8 und 9 zeigen die Kühlelemente aus Fig1 bis 3 bzw. 4 bis 6 nach dem Aufbringen auf einen Körper oder Körperteil an der, im Schnitt schematisch dargestellten, Hautoberfläche 7.In Fig7 wird dargestellt, dass sich in Kühlelementen, welche keine erfindungsgemäße Vorrichtung aufweisen, eine Flüssigkeitsschicht 8 bildet, welche die Wärmeübertragung zwischen dem festen Kühlkörper 2 und der Hautoberfläche 7 bereits nach kurzer Zeit signifikant verringert.
Fig8 und Fig9 zeigen zwei unterschiedliche Ausprägungen der erfindungsgemäßen Vorrichtung. In Fig. 8 wird dargestellt, dass nach der Erwärmung des Kühlkörpers, durch die Ausdehnung des Dehnkörpers 4, der noch feste Anteil des Kühlkörpers 2 an die Haut angepresst wird und die bereits geschmolzene Flüssigkeit 8 dadurch nach oben verdrängt wird. Fig. 9 zeigt eine Ausdehnung des mechanischen Dehnkörpers 6, welcher selektiven und gezielten Druck auf den noch festen Anteil des Kühlkörpers 2 ausübt und den bereits geschmolzenen Anteil 8 des Kühlkörpers an die der Hautoberfläche 7 abgewandte Seite verdrängt. Dadurch kann in beiden Fällen der direkte Kontakt des noch festen Anteils der Kühlkörper zur Hautoberfläche und damit die Wärmeleitung und die Effizienz der Kühlauflage erhalten bleiben.
Fig10 zeigt in der Aufsicht ein Beispiel wie mehrere Kühlelemente 11, in diesem Fall in quadratischer Form, zu einer Kühlauflage 10 kombiniert werden können. Fig11 zeigt die Kräfte in einem Kühlkörper während des Auftauvorgangs. Der Pfeil 13 symbolisiert dabei die Kraft, welche den noch festen Anteil des Kühlkörpers an die Gegenwand andrückt und dadurch den bereits flüssigen Anteil des Kühlkörpers an der Kontaktfläche zur Haut 7 entsprechend den Pfeilen 12 nach oben verdrängt. Fig12 zeigt ein Positionierungsbeispiel wie die Kühlauflage 10 auf einen Körper oder Körperteil 14 mittels einer Klebefolie oder durch mechanische Vorrichtungen wie Bandagen aufgebracht werden kann.

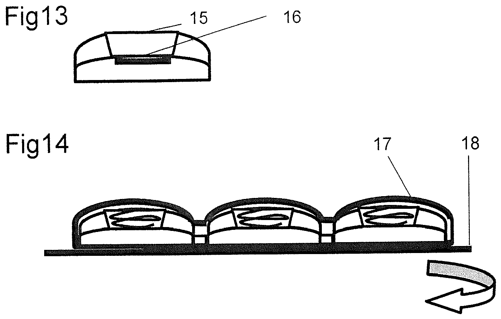

Fig13 zeigt im Schnitt die Positionierung einer Thermochromfarbschicht 16 welche eine genaue Indikation der Temperatur der Kühlauflage im Inneren ermöglicht. Durch die erfindungsgemäße Anbringung an der Unterseite des Dehnkörpers ist langfristig der Kontakt mit dem noch festen Anteil des Kühlkörpers gewährleistet, wodurch die Erreichung eines Temperatur-Grenzwertes, der das Absinken der Leistungsfähigkeit signalisiert, sehr genau durch Farbindikatoren angezeigt werden kann. Bei Verwendung einer transparenten Hülle des Dehnkörpers kann dieser Grenzwert von außen durch das dadurch gegebene Sichtfenster 15 festgestellt werden.
Fig14 zeigt eine formstabile Hülle 17 in welcher die Kühlauflage während des Einfriervorgangs zur Vermeidung der unkontrollierten Dehnung aufbewahrt wird. Diese Hülle kann gleichzeitig als Verpackung dienen. Die Unterschicht 18 kann zur Entnahme der Kühlauflage wie bei einer Blister-Verpackung entfernt werden.

## Patentansprüche

1. Vorrichtung zum Kühlen eines Körpers oder eines Körperteils für medizinische oder leistungssteigernde Zwecke, bestehend aus einem oder mehreren Kühlelementen (11), wobei das Kühlelement (11) eine Hülle (1) aufweist, welche mit einem schmelzbaren Kühlkörper (2) gefüllt ist, wobei in der Hülle (1) eine zusätzliche Vorrichtung zum Verdrängen des bereits geschmolzenen Anteils (8) des Kühlkörpers (2) von der Kontaktfläche zum Körper vorgesehen ist, **dadurch gekennzeichnet, dass** die Vorrichtung des weiteren eine formstabile Überhülle (17) aufweist, in der die Hülle (1) während des Einfrierens aufbewahrt werden kann, und wobei die Überhülle vorzugsweise als Verpackung dient.

2. Vorrichtung nach Anspruch 1, **dadurch gekennzeichnet, dass** die zusätzliche Vorrichtung als Dehnkörper (4, 6), vorzugsweise in Form eines gasgefüllten Körpers (4) eines Federelements (6) oder eines Schaumstoffkörpers ausgebildet ist.

3. Vorrichtung nach Anspruch 1, **dadurch gekennzeichnet, dass** das Überschreiten eines Temperaturschwellwertes durch eine an der Unterseite des Dehnkörpers aufgebrachte, und damit an den verbleibenden festen Anteil des Kühlkörpers angepresste Schicht (16) aus Thermochrommaterial angezeigt wird, wobei die Hülle (1) in diesem Fall transparent ausgeführt wird.

## Claims

1. An apparatus for cooling a body or body part for medical or performance-enhancing purposes, consisting of one or more cooling elements (11), wherein the cooling element (11) comprises a cover (1) which is filled with a meltable cooling solid (2), wherein an additional apparatus for displacing the already melted portion (8) of the cooling solid (2) from the contact surface (7) to the body is provided inside the cover (1), **characterised in that** the apparatus further comprises a dimensionally stable additional cover (17) in which the cover (1) can be stored during freezing and wherein the additional cover preferably serves as packaging.

2. An apparatus according to claim 1, **characterised in that** the additional apparatus is formed as an expandable body (4, 6), preferably in form of a gas-filled body (4) of a spring element (6) or a foamed article.

3. An apparatus according to claim 1, **characterised in that** the exceeding of a temperature threshold is indicated by a layer (16) made of thermochromic material which is applied to the bottom side of the expandable body and thus pressed against the remaining solid portion of the cooling solid, wherein the cover (1) is formed in a transparent manner in this case.

## Revendications

1. Dispositif de refroidissement d'un corps ou d'une partie d'un corps à des fins médicales ou pour améliorer les performances constitué par au moins un élément de refroidissement (11), cet élément de refroidissement (11) comprenant une gaine (1) qui est remplie d'un dissipateur thermique fusible (2), la gaine (1) renfermant un dispositif supplémentaire permettant de refouler la partie déjà fondue (8) du dissipateur thermique (2) de la surface de contact avec le corps,
**caractérisé en ce que**
le dispositif comporte en outre une surenveloppe (17) de forme stable dans laquelle la gaine (1) peut être maintenue pendant la congélation, la surenveloppe servant, de préférence, d'emballage.

2. Dispositif conforme à la revendication 1,
**caractérisé en ce que**
le dispositif supplémentaire est réalisé sous la forme d'un corps extensible (4, 6), de préférence sous la forme d'un corps rempli de gaz (4) d'un élément élastique (6) ou d'un corps en mousse.

3. Dispositif conforme à la revendication 1,
**caractérisé en ce que**
le dépassement d'une valeur de seuil de la température est indiqué par une couche (16) en un matériau thermo-chromé appliquée sur la face inférieure du corps extensible, et ainsi comprimée sur la partie solide restante du dissipateur thermique, la gaine (1) étant alors, transparente.
